Europäisches Patentamt

European Patent Office

Office européen des brevets

(19)

(11) **EP 0 937 773 B1**

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(51) Int Cl.[7]: **C12N 9/00**, C12C 1/047

(21) Anmeldenummer: **98120764.0**

(22) Anmeldetag: **02.11.1998**

(54) **Verfahren zur Herstellung von Enzymen bzw. Enzymkomplexen**

Process for the preparation of enzymes and enzyme complexes

Procédé pour la préparation des enzymes et complexes enzymatiques

(84) Benannte Vertragsstaaten:
**DE ES FR IT NL**

(30) Priorität: **28.01.1998 DE 19803070**

(43) Veröffentlichungstag der Anmeldung:
**25.08.1999 Patentblatt 1999/34**

(73) Patentinhaber: **Jodlbauer, Heinz D. Dr.**
**30161 Hannover (DE)**

(72) Erfinder: **Jodlbauer, Heinz D. Dr.**
**30161 Hannover (DE)**

(74) Vertreter:
**Hauck, Graalfs, Wehnert, Döring, Siemons**
**Neuer Wall 41**
**20354 Hamburg (DE)**

(56) Entgegenhaltungen:
**DE-A- 1 442 145**       **DE-A- 2 027 946**
**DE-A- 2 610 532**       **DE-B- 1 197 420**

**Beschreibung**

Die Erfindung bezieht sich auf ein Verfahren zur Herstellung von Enzymen bzw. Enzymkomplexen aus pflanzlichen Samen.

Sogenannte technische Enzyme in der Lebensmittel- und chemischen Industrie werden heutzutage mit Hilfe von Schimmelpilzen oder Bakterien gewonnen. Vielfach besteht der Wunsch bzw. die Notwendigkeit, auf "natürlicher" Basis produzierte Enzyme zu verwenden.

Derartige Enzyme sind an sich bekannt. Sie entstehen z. B. bei der Ankeimung von Samen und Getreide, etwa bei Mälzen von bestimmten Getreidearten für Brauereien und Brennereien. Gersten-, Weizen- und Roggenmalze werden in großen Mengen produziert und eingesetzt. Der Vermälzungsprozeß ist ein Zweistufenprozeß und besteht aus der Keimphase zur Herstellung des sogenannten Grünmalzes und dem sich anschließenden Darrprozeß, einem Röst- und Trocknungsprozeß. Das Ergebnis ist ein mehr oder weniger aromatisiertes Malzprodukt für die Bierherstellung mit unterschiedlichen Gehalten an Enzymen. Der Darrprozeß findet bei relativ hohen Temperaturen statt, beispielsweise bei Farbmalzen bis zu 105°C und hellen Malzen bis zu 85 °C. Die Folge ist, daß die im Keimprozeß gebildeten Enzyme teilweise bis vollständig denaturiert werden und somit ihre Wirksamkeit verloren geht.

Es ist jedoch auch bereits bekannt, ungedarrte trockene Cerealienmalze, wie beispielsweise sogenannte Luft- oder Diastasemalze herzustellen. Ziel dieses Verfahrens ist die höchstmögliche Anreicherung an Enzymkomplexen bei Einsatz von niedrigen Temperaturen.

Aus DE 14 17 568 ist ein Verfahren bekannt, an dem anstelle eines Darrprozesses eine Behandlung von Getreide mit gespanntem Wasserdampf oder durch direkte Erhitzung mit Heißdampf vorgesehen ist. Hierbei werden Temperaturen zwischen 180 und 250 °C angewendet. Trotz relativ kurzer Erhitzungszeiten steigen die Temperaturen im Keimgut bis auf 75 °C. Es treten jedoch nur geringe bzw. keine Verfärbungen im Trockenmalz auf. Gleichwohl wird auch in diesem Temperaturbereich ein großer Teil der Enzyme denaturiert.

Aus DE 20 27 946 ist ein Verfahren zur Behandlung von Gerste vor dem Mälzen beschrieben. Bei dem bekannten Verfahren wird das dem Keimling entfernt liegende Ende der Spelzen und Teile des Perikarps des Korns so abgeschliffen bzw. abgetragen, daß eine Wuchsstofflösung, z.B. Gibberellinsäure besser in das Korn eindringen kann, um so eine gewünschte Beschleunigung des Mälzprozesses herbeizuführen. Dadurch wird zwar eine Beschleunigung des Keimvorgangs erreicht, die ermälzte Enzymkonzentration bewegt sich jedoch im üblichen Rahmen. Die Abdarrtemperaturen sind ebenfalls im herkömmlichen Bereich.

Aus DE 14 42 145 ist ferner ein Verfahren zur Behandlung der Wurzelkeime und Sprößlinge im Tieftemperaturbereich beschrieben mit dem Ziel, diese zu inaktivieren, um so eine alpha-Amylasekonzentration bis etwa 65 D. E./g durch eine Gibberellinsäurebehandlung zu erreichen.

Die bisher bekannten Verfahren haben ausnahmslos den Mangel, daß die erzielbare Enzymkonzentration relativ gering ist. Der Einsatz von pflanzlichen Wuchsstoffen, wie z.B. Gibberelline, kann den Mangel nicht oder nur teilweise ausgleichen.

Der Erfindung liegt die Aufgabe zugrunde, pflanzliche Samen so zu behandeln, daß deutlich erhöhte Konzentrationen an Enzymen bzw. Enzymkomplexen erhalten wird.

Diese Aufgabe wird durch die Merkmale des Patentanspruchs 1 gelöst.

Liposome oder Niosome haben Biomembranen relativ leicht und rasch zu durchdringen. Liposome sind wäßrige Kompartimente, die von einer geschlossenen Lipid-Doppelschicht umgeben sind. Sie werden zum Beispiel dadurch erhalten, daß geeignete Lipide in wäßriger Lösung suspendiert werden, und die Mischung mit Ultraschall behandelt wird. Es entsteht eine Dispersion von ungefähr gleich großen geschlossenen Vesikeln. Solche Vesikel lassen sich aber auch dadurch erzeugen, daß man eine Lipidlösung rasch mit Wasser mischt. Wird das Lipid durch eine dünne Nadel in die wäßrige Lösung eingespritzt, entstehen runde Vesikel von z.B. 50 nm Durchmesser. Es sind jedoch auch chemisch-synthetische Liposombildner bekannt. Hier spricht man von sogenannten Niosomen.

Bei der Erfindung wurde nun erkannt, daß der Keimling eines Pflanzensamens, insbesondere das Schildchen zusammen mit dem sich vergrößernden Aufsaugepithel als chemischer Reaktor verwendet werden kann, indem eine Aktivierung durch Liposome oder Niosome erfolgt. Die pflanzlichen Samen werden wie bei einem Mälzprozeß einem herkömmlichen Weich- und Keimvorgang in wäßriger Umgebung ausgesetzt. Der wäßrigen Umgebung werden die die Biomembran durchwandernden Liposome oder Niosome zugesetzt. Dies führt zum einen zu einer erhöhten Aktivierung der sameneigenen Gibberelline. Andererseits wird das sameneigene Leitungsnetz benutzt, d.h. ein verstärkter Transport von Gibberellinen zur Eleuronschicht und ein Rücktransport von Reservestoffen aus dem Mehlkörper zum Keimling. Offensichtlich wirkt sich die Aktivierung der vergrößerten "Aufnahmeoberfläche des Aufsaugepithels" positiv aus. Dies hat durch die Aktivierung der Wuchsstoffe und den Transport zur Aleuronschicht mit dem Ziel, die Neubildung von Enzymen bzw. Enzymkomplexen zu verstärken, zur Folge, daß gezielt eine Anreicherung bestimmter Enzyme, wie beispielsweise Alpha-Amylase, Beta-Amylase, Beta-Glucanase, Pentosonase, Oxidase, Lipoxigenase, Phospholipase, Phosphatase, Lipase sowie spezielle Proteasen in Ganz gesetzt wird. Gleichzeitig kann bei dem beschriebenen Vorgang auch eine Unterdrückung von bestimmten Enzymen und Enzymkomplexen stattfinden. Insgesamt spielt das Verhältnis von Länge des Keimlings zu Länge des Mehlkörpers verbunden mit dem Auflösungsgrad (Stärkeauflösung) eine nicht unerhebliche Rolle.

Die Liposome oder Niosome bilden einen Trägerstoff, der den Transport im Inneren des Pflanzensamens fördert, nachdem er in diesen eingedrungen ist. Wie sich gezeigt hat, wird dadurch die Enzymkonzentration im gekeimten Gut auf eine bisher nicht bekannte Höhe gebracht.

Nach einer Ausgestaltung der Erfindung können die Liposome aus Lecithinen bzw. Lecithinfraktionen hergestellt sein. Beispielsweise kann ein Lecithin verwendet werden, das vorwiegend Phosphatidylcholin (PC) enthält. Alternativ kann eine Lecithinfraktion aus Phosphatidylethanolamin und Phosphatidylinositol (PE bzw. PI) verwendet werden. Werden Niosome eingesetzt, sieht eine Ausgestaltung der Erfindung vor, daß hierfür ein Alkylpolyglycerinether zusammen mit Cholesterin und Diacetylphosphat als Niosombildner verwendet wird oder ein Glycero-lactyl-palmitat.

Die Vesikel der verwendeten Liposome oder Niosome liegen nach einer Ausgestaltung der Erfindung im Größenbereich von 10 bis 3000 nm, vorzugsweise zwischen 50 und 200 nm.

Bei einer anderen Ausgestaltung der Erfindung ist die Substanz mit einer die Enzymbildung fördernden Beladungssubstanz beladen. Das Beladen von Leerliposomen bzw. -niosomen mit einem Wirkstoff ist an sich bekannt und erfolgt nach den in der Industrie bekannten Verfahren. Die Art des Wirkstoffes ist von entscheidender Bedeutung. Je nachdem ob es sich um lipophile, hydrophobe oder hydrophile Stoffe handelt, findet die Beladung in der Hülle des Liposoms oder im Innenraum statt. Wirkstoffe mit lipophilem oder hydrophobem Charakter, wie zum Beispiel Indolyl-3-Essigsäure (IAA) oder Gibberellinsäure (GA) werden in die Hülle (auch lipophile Doppelschicht oder Bilayer-Membrane genannt), hydrophile Substanzen, wie zum Beispiel Ionen oder Salzlösungen, werden im Innenraum eingeschlossen und gelangen ohne Schwierigkeiten in die vorgesehenen Zentren.

Bei dem erfindungsgemäßen Verfahren kann nach einer Ausgestaltung der Erfindung eine Reihe von Stoffen als Beladungssubstanz eingesetzt werden, wie ein-, zwei- oder mehrwertige Ionen, wie Chloride, Phosphate, Ammonium, Kalium, Calcium, Magnesium, Kupfer, Mangan und/oder Eisen, organische Säuren, wie zum Beispiel Ascorbinsäure, Essigsäure, Aminosäuren. Als Aminosäuren können zum Beispiel Tryptophan, Milchsäure, Orotsäure, Guanin, Guanidin, Adenosin-5-tri-phosphat Dinatrium (ATP) sowie Wuchsstoffe (auch als Wachstumsregulatoren bezeichnet), wie Gibberelline (GA) und Abscisinsäure (ABA) allein oder in Kombination der Stoffe verwendet werden. Bei Liposomlösungen liegt der durchschnittliche Durchmesser der Liposome vorzugsweise im Bereich von 100 nm. Es können nicht nur Mono- und Bilayer-Strukturen verwendet werden, sondern auch gemischte Strukturen (Multilayer-Strukturen). Erfolg eine Beladung mit einem Wirkstoff, dient das Liposom als Transportmedium zu den aktiven Keimzentren bzw. zu den aktiven Enzymzentren im biologischen Material. Je nach Polarität der Beladungssubstanz erfolgt die Einkapselung im Bilayerbereich, d.h. in den lipophilen Teil oder in den hydrophilen Teil des Vesikels, also im Kugelinneren. Eine Besonderheit ist die Einkapselung von technischen Enzymen, wie z.B. Cellulase oder Hemicellulase, um z.B. die äußeren Schichten des Samenkorns teilweise abzubauen. Dadurch kann ein schnelleres Eindringen der Wirksubstanz in das Innere des Sämlings erhalten werden.

Die Behandlungsphase, d.h. das Aussetzen der Sämlinge dem Wasser und den Liposomen oder Niosomen findet nach einer Ausgestaltung der Erfindung über einen Zeitraum von 1 bis 12 Stunden statt. Die Weichphase beträgt vorzugsweise 1 bis 6 Stunden. Die Keimphase beträgt 2 bis 10 Tage, vorzugsweise 3 bis 4 Tage. Die in der Keimphase herrschende Temperatur wird vorzugsweise gesteuert und auf relativ niedrigen Werten gehalten, etwa zwischen 10 und 25 °C, vorzugsweise 14 bis 15 °C. Zu diesem Zweck erfolgt üblicherweise eine ständige Kühlung, die vorzugsweise mit Luft stattfindet.

Nach einer vorgegebenen Dauer wird die Keimphase unterbrochen. Hierzu sieht die Erfindung vor, daß die Unterbrechung durch ein Schockgefrieren stattfindet. Hierzu wird eine Temperatur von -10 °C bis -40 °C, vorzugsweise von -16 °C angewendet. Nach Beendigung der Keimphase erfolgt vorzugsweise ein Trocknen des Keimgutes. Hierzu kann das an sich bekannte Gefriertrocknen eingesetzt werden. Nach einer Ausgestaltung der Erfindung wird das Gut auf einen Wassergehalt von 3 bis 10 %, vorzugsweise von 6 % eingestellt.

Zur Gewinnung der Enzyme bzw. des Enzymkomplexes aus dem Keimgut wird dieses vorzugsweise gemahlen. Das Mahlgut kann dann separiert werden in eine enzymreiche und proteinreiche Fraktion und eine proteinarme und enzymarme Fraktion. Dies geschieht vorzugsweise durch Windsichtung. Derartige Verfahren sind an sich bekannt.

Eine Liposomlösung wird etwa nach folgender Rezeptur hergestellt:

| | |
|---|---|
| Phospholipidfraktion (mit 40 % Phosphatidylcholingehalt) | 5,000 |
| Wasser | 45,000 |
| | 50,000 |

Zur Anwendung wird die Liposomlösung etwa wie folgt verdünnt:

|  | kg |
|---|---|
| Liposomlösung | 50,000 |
| Wasser | 100,000 |
|  | 150,000 |

Eine weitere Verdünnung kann nach dem nachstehenden Beispiel erfolgen:

| 1. Verdünnungslösung | 150,000 |
|---|---|
| Wasser | 7 500,000 |
|  | 7 650,000 |

Bei diesem Beispiel enthält die Endliposomlösung 0,065 % Liposom.

Die oben angegebenen Zahlen sind Gew.-%. Dies trifft auch auf die nachstehenden Beispiele zu.

Die beschriebene Liposomlösung kann z.B. zur Behandlung von Weizen oder Sojabohnen verwendet werden.

Nachstehend ein Beispiel zur Behandlung von Weizen über eine Dauer von 1 bis 12 Stunden:

|  | kg |
|---|---|
| Weizen | 3,000 |
| Endliposomlösung | 2,250 |
| Gesamteinsatzmenge | 5,250 |
|  |  |
| ./. nicht aufgenommene Liposomlösung | 1,480 |
| gesamte eingesetzte Liposomlösung | 2,250 |
|  |  |
| Vom Weizen aufgenommene Liposomlösung | 1,770 |

Weichphase

Behandlungsdauer zwischen 1 bis 6 Stunden

| Weizen + Liposom | 4,770 |
|---|---|
| je nach Weichbedingungen bis zu 1,000 kg Wasser zum Wiederbefeuchten in der Keimphase | 1,000 |
|  | 5,770 |

Keimphase

Dauer zwischen 2 bis 10 Tage, vorzugsweise um 3-4 Tage je nach erwünschtem Enzymprofil. Temperaturbereiche zwischen 10 °C bis 25 °C, vorzugsweise zwischen 14 °C bis 15 °C. Keimbedingungen: übliche Keimkästen, aber auch Trommelmalzanlagen können eingesetzt werden.

Kühlphase

Die Kühlphase erstreckt sich über alle 3 Phasen, wobei die erwünschte Leittemperatur zur Erzielung des erwünschten Enzymprofils über die gekühlte Luftmenge gesteuert wird.

Gefrierphase

Sind die erwünschten Enzymprofile im Keimgut erreicht, erfolgt der Einsatz der Gefrierphase zur Unterbrechung der Keimphase bei 10 °C bis -40 °C, vorzugsweise bei -16 °C in Form einer Schockfrostung, um so eine weitere Veränderung des produzierten Enzymprofils zu verhindern.

Trocknungsphase

Der Gefrierphase schließt sich zur Herstellung von stabilen Trockenprodukten die Trocknungsphase an. Dabei kann, um die Enzymverluste zu vermeiden, nicht die herkömmliche Darretrocknung eingesetzt werden, sondern es wird eine der schonungsvollsten Trockenmethoden, wie beispielsweise die Gefriertrocknung, angewandt, um so die gebildeten Enzyme weitestgehend im Trockengut zu erhalten. Das gefriergetrocknete Produkt wird auf einen Wassergehalt von ca. 6 %, vorzugsweise zwischen 3 und 10 % eingestellt.

Die Verwendung des ungetrockneten, tiefgefrorenen Keimgutes ist gleichermaßen denkbar, beispielsweise zum direkten Einsatz in Großbäckereien bzw. in der Lebensmittelindustrie. Zur Stabilisierung und Haltbarmachung kann zwecks Absenkung des pH-Wertes eine Beimpfung des Keimgutes mit Lactobazillenkulturen durchgeführt werden. Die Bakterienkultur wird in die Liposomlösung vorzugsweise während der Herstellungsphase eingebracht. Durch entsprechende Säurezugabe, vorzugsweise in der beginnenden Keimphase, wird die Tätigkeit der Milchsäurebakterien maßgeblich unterstützt.

Enzymprofile spielen bei derzeitigen Backmitteln, insbesondere für den Bereich der Gärunterbrechung in den Bäckereien, eine bedeutende Rolle und ergänzen die Funktion von chemisch-synthetischen Emulgatoren. Bisher wurden hierzu ausschließlich chemisch-synthetische Enzyme, sogenannte technische Enzyme, verwendet. Mit Hilfe des erfindungsgemäßen Verfahrens lassen sich auf pflanzlicher Basis die gewünschten Enzyme und Enzymprofile erzeugen, wobei ihr Einsatz nicht nur in der Lebensmittelindustrie, sondern auch in der chemischen Industrie vorteilhaft ist. Durch "Induktion" des Keimapparates, beispielsweise in Getreiden oder Leguminosen, werden spezifische Enzymprofile erzeugt, wobei alle an sich benötigten Enzyme in pflanzlichen Samen vorkommen. Nachstehend werden vier Beispiele für die Erzeugung von Enzymen nach der Erfindung angegeben und anderweitig hergestellten Enzymkonzentrationen gegenüber gestellt.

Tabelle 1

| WEIZEN Enzymprofil | | | |
|---|---|---|---|
| | Alpha-Amylase U/g TrS | Lipoxygenase U/g TrS pH 9,0 | Phosphorlipase C mmol/min/g |
| Weizengrünmalz, 6 Tage Keimung, ohne Erhitzung | 440 | 25870 | 18,5 |
| Weizendarrmalz, 6 Tage Keimung, maximale Darrtemperatur ca. 80 °C | 63 | 929 | 7,9 |
| Weizen nach Liposombehandlung keimen gelassen, danach gefriergetrocknet + Keime | 1223 | 60021 | 34,3 |

Tabelle 2

| SOJABOHNEN Enzymprofil | | |
|---|---|---|
| | Alpha-Amylase U/g TrS | Lipoxygenase U/g TrS pH 9,0 |
| Keimung 6 Tage ohne Erhitzung | 5,7 | 1476651 |
| Keimung 6 Tage mit Liposombehandlung gefriergetrocknet + Keime | 4,8 | 4330080 |

Tabelle 3

| ALPHA-AMYLASE Gehalte U/g TrS | | | |
|---|---|---|---|
| | Gerste | Roggen | Bohnen |
| ungekeimt | 0,8 | 4,1 | 2,3 |
| Keimung 3 Tage mit Liposombehandlung gefriergetrocknet | 3530 | 2080 | 5,0 |

Tabelle 4

| ACKERBOHNEN | | | |
|---|---|---|---|
| | Alpha-Amylase U/gTrS | Lipoxygenase U/gTrS pH 6,0 | Lipase U/gTrS |
| Keimung 8 Tage, mit Liposombehandlung, 0,05 molar an CaCl-Ionen gefriergetrocknet | 2,51 | 4070 | 32,90 |
| Keimung 8 Tage mit Liposombehandlung 0,05 molar an MgCl-Ionen gefriergetrocknet | 0,96 | 6330 | 58,50 |

In obigen Tabellen ist die erfindungsgemäße Anreicherung von spezielle Enzymprofilen aufgezeigt. Mit Hilfe der Erfindung ist es daher möglich, bisher noch nie erreichte Enzymkonzentrationen in pflanzlichen Materialien zu erreichen.

Nachstehend ein Verfahrensbeispiel als Großversuch:

Versuchsmenge: 2000 kg - nach herkömmlicher Art gereinigter - Weizen
Versuchsdauer: 6 Tage

Versuchsanlage (Pilotanlage):

Keimkästen (Saladinkästen Eigenkonstruktion), Fassungskapazität:
36 t mit Zonenregulierung, herkömmliche Kühlung (Kälteverdampfer); die Belüftung erfolgt mittels Ventilatoren, die minimale Beladungskapazität liegt bei ca. 1 t. Das Wendesystem besteht aus Wenderwagen und Wendespiralen; bis zu 5 t Beladungsmenge wurde manuell gewendet.
Liposombehandlung (gemäß vorangegangener Beschreibung) + Weiche: 12 Stunden. Die Behandlung führte zu einer Temperatursteigerung von 5,5 °C (Einweichtemperatur 9,7 °C, Ausweichtemperatur 15,2 °C).

Temperaturmessung zu Beginn des Keimversuches

Temperatur am Geräteschrank: T°

über der Horde: 16,5 °C
unter der Horde: 15,0 °C
Rückluft: 100 % bis 95 %
Frischluft: 0 %

Temperaturverlauf:

| Keimtage | Temperatur °C, im Gut direkt gemessen morgens-mittags-abends | | |
|---|---|---|---|
| 1. Tag | 15,3 | 14,5 | 17,0 |

(fortgesetzt)

| Keimtage | Temperatur °C, im Gut direkt gemessen morgens-mittags-abends | | |
|---|---|---|---|
| 2. Tag | 14,7 | 13,4 | 13.5 |
| 3. Tag | 13,9 | 13,7 | 14,2 |

Wassergehalt im Keimgut nach 3. Tag: 39,1 %

Temperatur am Geräteschrank nach dem 3. Tag: T°

über der Horde : 16,0 °C
unter der Horde: 14,5 °C
Rückluft: 94 %
Frischluft: 22%

| 4. Tag | 13,5 | 13.6 | 14,1 |
|---|---|---|---|
| 5. Tag | 13,9 | 14,7 | 14,9 |

Temperatur am Geräteschrank nach dem 5 Tag: T°

über der Horde : 16,9 °C
unter der Horde: 16,5 °C
Rückluft: 95 %
Frischluft: 22%

| 6. Tag | 14,7 | 15,2 | 15,1 |
|---|---|---|---|

Die laufende Befeuchtung des Keimgutes erfolgte manuell nach dem Gießkannenprinzip unter laufender Berücksichtigung der praktischen Mälzererfahrung (Tennenmälzereierfahrung).

Das so erhaltene Keimgut wird sofort nach herkömmlichen, bekannten Verfahren tiefgefroren und der Gefriertrocknung zugeführt.

Der Keimversuch wird über die Temperatur im Keimgut gesteuert. Besondere Berücksichtigung erfährt der Temperatursprung nach der Liposombehandlung.

Die Wirksamkeit der Liposombehandlung im Rahmen der 6-tägigen Keimung wird über die Bestimmung des Enzymprofils mittels chemischer Analyse sowie eines Standardbackversuches (RMT-Backversuches) ermittelt.

Der Großversuch ergab folgende Werte für das Enzymprofil:

Chemische Analyse:

| | Alpha-Amylase U/g TrS | Lipoxygenase U/g TrS pH 6,0/9,0 | Phosphorlipase C mmol/min/g |
|---|---|---|---|
| Weizen nach Liposombehandlung 6 Tage keimen gelassen, eingefroren und gefriergetrocknet + Keime | 1039 | 1498/9435 | 30,2 |

Backversuch abgewandelt nach RMT-Standardbackversuch

Rezeptur:

| 1000 g | Mehl Type 550 |
|---|---|
| 550 g | Wasser |
| 50 g | Hefe |
| 20 g | Salz |
| 10 g | Zucker |
| 10 g | Biskin |

Teigtemperatur: 25-26 °C

Ergebnis:

|  | 0-Versuch nur Mehl | mit Enzympräparat incl. Keime Zugabemenge: 5 g + 995 g Mehl = 0,5 % |
|---|---|---|
| Backvolumen (ml) | Normalgare: 3250 | Normalgare: 3790 |
|  | Übergare: 3030 | Übergare: 3670 |

Erfindungsgemäß hergestellte Enzymprofile können in unterschiedlichen Konzentrationen in herkömmlichen Backmittelrezepturen, die bisher auf der Basis chemischsynthetischer Emulgatoren erzeugt worden sind, eingesetzt werden. Vorzugsweise werden sie bei Backmittelrezepturen verwendet, die als Emulgatoren Lecithine bzw. Lecithinfraktionen enthalten. Bei diesen werden die in üblichen Backmittelrezepturen verwendeten technischen Enzyme vollständig durch erfindungsgemäße Enzymprofile in Form von Mischpräparaten unterschiedlicher Pflanzenmaterialien ersetzt. Eine derartige Backmittelrezeptur sei in einem nachfolgenden Beispiel angegeben:

Typenbezeichnung AKH 156

|  | % |
|---|---|
| Guarkernmehl | 20,000 |
| Dextrose | 20,000 |
| Saccharose | 10,000 |
| Ascorbinsäure | 0,250 |
| IM10 | 15,000 |
| UM 20 | 3,750 |
| LC 2000 (30%) | 30,000 |
| ES 1000 | 1,000 |
|  | 100,000 |

IM 10: Weizen, nach Liposombehandlung 6 Tage keimen gelassen, gefriergetrocknet, gemahlen und zum Teil windgesichtet mit Enzymprofil ,wie bereits erfindungsgemäß beschrieben.

UM 20: Wintergerste, nach Liposombehandlung 3 Tage keimen gelassen, gefriergetrocknet und gemahlen mit Enzymprofil, wie bereits erfindungsgemäß beschrieben.

ES 1000: Sojabohne, nach Liposombehandlung 6 Tage keimen gelassen, gefriergetrocknet und gemahlen, zum Teil windgesichtet mit Enzymprofil, wie bereits erfindungsgemäß beschrieben, Verdünnung 1 : 10000.

LC 2000. 30 %iges Lecithingemisch auf der Basis Weizenmehl; bei dem Lecithin handelt es sich um eine Lecithinfraktion gemäß EP 0 245 723 B1.

Die obige beispielhafte Backmittelrezeptur wurde verbacken und mit einem auf dem deutschen Markt vertriebenen Produkt mit dem Handelsnamen Goldmalz verglichen. Aus dem nachfolgenden Backprotokoll sind die Backergebnisse dargelegt. Daraus ergibt sich, daß bei einem Enzymprofil, das nach dem erfindungsgemäßen Verfahren erzeugt worden ist, zu herkömmlichen Enzymen äquivalente Backleistungen erbracht werden können, vorzugsweise im Tiefkühlbereich bei der Gärverzögerung und der Gärunterbrechung.

Backprotokoll :

## Rapid - Mix - Test

Anlage 1

Datum: 21.04.1997

| Musterbezeichnung | AHK-156 | | Goldmalz | |
|---|---|---|---|---|
| Mehlmenge (g) (Roland 550) | 1000 | | 1000 | |
| Flüssigkeit (ml) | 590 | | 590 | |
| Hefe (g) | 50 | | 50 | |
| Salz (g) | 15 | | 15 | |
| Backmittel (g) | 25 | | 25 | |
| Fett P 1 (g) | 10 | | 10 | |
| Erdnußfett (g) | - | | | |
| Zucker (g) | - | | | |
| Temperatur des Teiges sofort (°C) | 25,8 | | 25,7 | |
| Teiggewicht | 1693 | | 1683 | |
| Teigbeschaffenheit  Oberfläche | normal | | normal | |
| Elastizität | normal | | geschmeidig | |
| Stückgärzeit (min) | 25 | 30 | 25 | 30 |
| Teiggewicht (g)  (15 auf 30 umgerechnet) | 1688 | 1692 | 1690 | 1678 |
| Gebäckgewicht (g) (15 auf 30 umgerechnet) | 1284 | 1290 | 1278 | 1284 |
| Backverlust (%) | 23,9 | 23,2 | 24,4 | 23,4 |
| Gebäckvolumen Ausbund (cm³), mind. 15 | 6800 | - | - | - |
| Gebäckvolumen Presse gesamt (cm³) | 6800 | 6780 | 6550 | 6600 |
| Ausbund  Note | gut | noch gut | noch gut | mangelhaft |
| Art | - | et. schmal | et. schmal | alle n. ausgeb. |
| Bräunung | normal | normal | normal | normal |
| Rösche  Note | gut | gut | gut | noch gut |
| Art | - | - | - | et. weichsplittrig |
| Porengleichmäßigkeit | zieml. glm. | glm. | glm. | zieml. glm. |
| Krumenelastizität | noch gut | noch gut | noch gut | noch gut |
| Geschmack | einwandfrei | einwandfrei | einwandfrei | einwandfrei |
| Brötchen Ausbund/Schneider | 15/0 | 8/7 | 8/7 | 0/15 |
| Bemerkungen | | | Zum Teil nicht maschinengängig (6 von 30 Teiglingen) | |

**Patentansprüche**

1. Verfahren zur Herstellung von Enzymen bzw. Enzymkomplexen, **gekennzeichnet durch** folgende Verfahrensschritte:

   Pflanzliche Samen werden einem Weich- und Keimvorgang in wäßriger Umgebung ausgesetzt und

   der wäßrigen Umgebung werden zumindest vor und während des Weichvorgangs Liposome oder Niosome zugesetzt.

2. Verfahren nach Anspruch 1, **dadurch gekennzeichnet, daß** die Liposome aus Lecithin bzw. einer Lecithin-Fraktionen hergestellt sind.

3. Verfahren nach Anspruch 2, **dadurch gekennzeichnet, daß** das Lecithin vorwiegend Phosphatidylcholin enthält.

4. Verfahren nach Anspruch 2, **dadurch gekennzeichnet, daß** eine Phosphatidylethanolamin und Phosphatidylinositol enthaltende LecithinFraktion verwendet wird.

5. Verfahren nach Anspruch 1, **dadurch gekennzeichnet, daß** ein Alkylpolyglycerinether zusammen mit Cholesterin und Diacetylphosphat oder ein Glycero-lactyl-palmitat zur Niosombildung verwendet wird.

6. Verfahren nach einem der Ansprüche 1 bis 5, **dadurch gekennzeichnet, daß** die Vesikel in der Größenordnung von 10 bis 300 nm, vorzugsweise zwischen 50 und 200 nm, liegen.

7. Verfahren nach einem der Ansprüche 1 bis 6, **dadurch gekennzeichnet, daß** die Substanz mit einer die Enzymbildung fördernden Beladung zur Substanz beladen wird.

8. Verfahren nach Anspruch 7, **dadurch gekennzeichnet**, da als Beladungssubstanz ein-, zwei- oder mehrwertige Ionen vorgesehen sind, wie Chloride, Phosphate, Ammonium, Kalium, Calcium, Magnesium, Kupfer, Mangan und/oder Eisen.

9. Verfahren nach Anspruch 7, **dadurch gekennzeichnet, daß** als Beladungssubstanz organische Säuren vorgesehen sind, wie Ascorbinsäure, Essigsäure und/oder Aminosäuren.

10. Verfahren nach Anspruch 9, **dadurch gekennzeichnet**, als Aminosäuren Tryptophan, Milchsäure, Orotsäure, Guanin, Guanidin oder Adenosin-5-tri-phosphat Dinatrium vorgesehen sind.

11. Verfahren nach Anspruch 8, **dadurch gekennzeichnet, daß** Wuchsstoffe als Beladungssubstanz vorgesehen sind.

12. Verfahren nach Anspruch 11, **dadurch gekennzeichnet, daß** als Wuchsstoffe Gibberelline, Abscisinsäure oder eine Kombination von beiden vorgesehen sind.

13. Verfahren nach einem der Ansprüche 1 bis 12, **dadurch gekennzeichnet, daß** die Behandlungsphase mit der Substanz 1 bis 12 Stunden beträgt.

14. Verfahren nach einem der Ansprüche 1 bis 13, **dadurch gekennzeichnet, daß** die Weichphase 1 bis 6 Stunden beträgt.

15. Verfahren nach einem der Ansprüche 1 bis 13, **dadurch gekennzeichnet, daß** die Keimphase 2 bis 10 Tage, vorzugsweise 3 bis 4 Tage, beträgt.

16. Verfahren nach einem der Ansprüche 1 bis 15, **dadurch gekennzeichnet, daß** während der Keimphase eine Temperatur von 10 bis 25°C vorgesehen ist, vorzugsweise von 14 bis 15°C.

17. Verfahren nach einem der Ansprüche 1 bis 16, **dadurch gekennzeichnet, daß** während der einzelnen Phasen gekühlt wird.

**18.** Verfahren nach Anspruch 17, **dadurch gekennzeichnet, daß** mit Luft gekühlt wird.

**19.** Verfahren nach einem der Ansprüche 1 bis 18, **dadurch gekennzeichnet, daß** nach Ablauf einer vorgegebenen Dauer die Keimphase diese unterbrochen wird.

**20.** Verfahren nach Anspruch 19, **dadurch gekennzeichnet, daß** die Unterbrechung durch Schockgefrieren vorgenommen wird.

**21.** Verfahren nach Anspruch 20, **dadurch gekennzeichnet, daß** das Schockgefrieren bei -10°C bis -40°C, vorzugsweise bei -16°C, durchgeführt wird.

**22.** Verfahren nach einem der Ansprüche 1 bis 21, **dadurch gekennzeichnet, daß** nach der Beendigung der Keimphase eine Trocknungsphase eingeleitet wird.

**23.** Verfahren nach Anspruch 22, **dadurch gekennzeichnet, daß** eine Gefriertrocknung erfolgt.

**24.** Verfahren nach Anspruch 22 oder 23, **dadurch gekennzeichnet, daß** auf einen Wassergehalt von 3 bis 10 %, vorzugsweise von 6%, eingestellt wird.

**25.** Verfahren nach einem der Ansprüche 1 bis 24, **dadurch gekennzeichnet, daß** das Keimgut nach der Trocknung gemahlen wird.

**26.** Verfahren nach Anspruch 25, **dadurch gekennzeichnet, daß** das Mahlgut in zwei Fraktionen aufgeteilt wird, vorzugsweise durch Windsichtung, nämlich in eine proteinreiche enzymreiche Fraktion und eine proteinarme und enzymarme Fraktion.

**Claims**

**1.** Process for the preparation of enzymes and enzyme complexes, **characterized by** the following procedures:

Vegetable seeds are exposed to a softening and sprouting process in an aqueous environment and

liposomes or niosomes are added to the aqueous environment at least before and during the softening process.

**2.** Process according to Claim 1, **characterized in that** the liposomes are prepared from lecithin or a lecithin fraction.

**3.** Process according to Claim 2, **characterized in that** the lecithin contains predominantly phosphatidylcholin.

**4.** Process according to Claim 2, **characterized in that** a lecithin fraction containing phosphatidylethanolamine and phosphatidylinositol is used.

**5.** Method according to Claim 1, **characterized in that** an alkyl polyglycerin ether combined with cholesterol and diacetyl phosphate or a glycero-lactyl-palmitate is used for niosome formation.

**6.** Process according to one of the Claims 1 to 5, **characterized in that** the vesicles are within the size magnitude of 10 to 300 nm, preferably between 50 and 200 nm.

**7.** Process according to one of the Claims 1 to 6, **characterized in that** the substance is charged with one of the enzyme boosting charges for the substance.

**8.** Process according to Claim 7, **characterized in that** univalent, bivalent or polyvalent ions, such as chloride, phosphate, ammonium, potassium, calcium, magnesium, copper, manganese and/or iron, are provided as a charging substance.

**9.** Process according to Claim 7, **characterized in that** organic acids, such as ascorbic acid, ascetic acid and/or amino acid are provided as a charging substance.

**10.** Process according to Claim 9, **characterized in that** tryptophan, lactic acid, orotic acid, guanine, guanidine or adenosin-5-tri-phosphate dinatrium are provided as amino acids.

**11.** Process according to Claim 8, **characterized in that** growth promoters are provided as a charging substance.

**12.** Process according to Claim 11, **characterized in that** gibberellin, abscisic acid or a combination of the two are provided as growth promoters.

**13.** Process according to one of the Claims 1 to 12, **characterized in that** the treatment phase with the substance is 1 to 12 hours.

**14.** Process according to one of the Claims 1 to 13, **characterized in that** the softening phase is 1 to 6 hours.

**15.** Process according to one of the Claims 1 to 13, **characterized in that** the sprouting phase is 2 to 10 days, preferably 3 to 4 days.

**16.** Process according to one of the Claims I to 15, **characterized in that** during the sprouting phase a temperature of 10 to 25 °C, preferably from 14 to 15 °C, is provided.

**17.** Process according to one of the Claims 1 to 16, **characterized in that** cooling takes place during the individual phases.

**18.** Process according to Claim 17, **characterized in that** cooling is done with air.

**19.** Process according to one of the Claims 1 to 18, **characterized in that** the sprouting phase is interrupted after the completion of a prescribed duration.

**20.** Process according to Claim 19, **characterized in that** the interruption is done through quick-freezing.

**21.** Process according to Claim 20, **characterized in that** the quick-freezing is carried out at -10°C to -40 °C, preferably at -16 °C.

**22.** Process according to one of the Claims 1 to 21, **characterized in that** after the completion of the sprouting phase a drying phase is started.

**23.** Process according to Claim 22, **characterized in that** a freeze drying is done.

**24.** Process according to Claim 22 or 23, **characterized in that** the water content is set to 3 to 10 %, preferably 6 %.

**25.** Process according to one of the Claims 1 to 24, **characterized in that** the grist is ground after drying.

**26.** Process according to Claim 25, **characterized in that** the grist is separated into two fractions, preferably by air classification, specifically into a protein rich, enzyme rich fraction and a protein poor and enzyme poor fraction.

**Revendications**

**1.** Procédé de préparation des enzymes et complexes enzymatiques, **caractérisé par** les étapes de procédé suivantes :

Des semences végétales sont soumises à un processus de ramollissement et de germination dans un environnement aqueux et,

à l'environnement aqueux, sont ajoutés des liposomes ou des niosomes au moins avant et pendant le processus de ramollissement.

**2.** Procédé selon la revendication 1, **caractérisé en ce que** les liposomes sont préparés à partir de lécithine ou d'une fraction de lécithine.

**3.** Procédé selon la revendication 2, **caractérisé en ce que** la lécithine contient principalement de la phosphatidylcholine.

**4.** Procédé selon la revendication 2, **caractérisé en ce qu'**une fraction de lécithine contenant de la phosphatidyléthanolamine et du phosphatidylinositol est utilisée.

**5.** Procédé selon la revendication 1, **caractérisé en ce qu'**un alkylpolyglycérinéther en même temps que de la cholestérine et du diacétylphosphate ou un glycéro-lactyl-palmitat est utilisé pour la formation des niosomes.

**6.** Procédé selon une des revendications 1 à 5, **caractérisé en ce que** les vésicules s'inscrivent dans un ordre de grandeur de 10 à 300 nm, de préférence entre 50 et 200 nm.

**7.** Procédé selon une des revendications 1 à 6, **caractérisé en ce que** la substance est chargée avec une charge de substance favorisant la formation enzymatique.

**8.** Procédé selon la revendication 7, **caractérisé en ce que**, comme substance de charge, il est prévu des ions monovalents, bivalents ou polyvalents, tels que des chlorures, des phosphates, de l'ammonium, du potassium, du calcium, du magnésium, du cuivre, du manganèse et/ou du fer.

**9.** Procédé selon la revendication 7, **caractérisé en ce que**, comme substance de charge, il est prévu des acides organiques, tels que l'acide ascorbique, l'acide acétique et/ou des acides aminés.

**10.** Procédé selon la revendication 9, **caractérisé en ce que**, comme acides aminés, il est prévu du tryptophane, de l'acide lactique, de l'acide orotique, de la guanine, de la guanidine ou de l'adénosine-5-triphosphate disodique.

**11.** Procédé selon la revendication 8, **caractérisé en ce que** des substances de croissance sont prévues comme substance de charge.

**12.** Procédé selon la revendication 11, **caractérisé en ce que**, comme substances de croissance, il est prévu de la gibbérelline, de l'acide abscisique ou une combinaison des deux.

**13.** Procédé selon une des revendications 1 à 12, **caractérisé en ce que** la phase de traitement avec la substance prend 1 à 12 heures.

**14.** Procédé selon une des revendications 1 à 13, **caractérisé en ce que** la phase de ramollissement prend 1 à 6 heures.

**15.** Procédé selon une des revendications 1 à 13, **caractérisé en ce que** la phase de germination prend 2 à 10 jours, de préférence 3 à 4 jours.

**16.** Procédé selon une des revendications 1 à 15, **caractérisé en ce que**, pendant la phase de germination, il est prévu une température de 10 à 25 °C, de préférence de 14 à 15 °C.

**17.** Procédé selon une des revendications 1 à 16, **caractérisé en ce qu'**il y a un refroidissement pendant les différentes phases.

**18.** Procédé selon la revendication 17, **caractérisé en ce que** le refroidissement est effectué avec de l'air.

**19.** Procédé selon une des revendications 1 à 18,
**caractérisé en ce que**, à l'expiration d'un délai prédéfini, la phase de germination est interrompue.

**20.** Procédé selon la revendication 19, **caractérisé en ce que** l'interruption est effectuée par congélation ultrarapide.

**21.** Procédé selon la revendication 20, **caractérisé en ce que** la congélation ultrarapide est effectuée à une température de -10 °C à -40 °C, de préférence à -16 °C.

**22.** Procédé selon une des revendications 1 à 21, **caractérisé en ce que**, une fois la phase de germination achevée, une phase de séchage est lancée.

**23.** Procédé selon la revendication 22, **caractérisé en ce qu'**il se produit une lyophilisation.

**24.** Procédé selon la revendication 22 ou 23, **caractérisé en ce que** le réglage se fait sur une teneur en eau de 3 à 10%, de préférence de 6 %.

**25.** Procédé selon une des revendications 1 à 24, **caractérisé en ce que** la matière germée est moulue après le séchage.

**26.** Procédé selon la revendication 25, **caractérisé en ce que** la matière moulue est répartie en deux fractions, de préférence par triage à vent, à savoir en une fraction riche en protéines et riche en enzymes, et en une fraction pauvre en protéines et pauvre en enzymes.